**Europäisches Patentamt**

(19) **European Patent Office**

**Office européen des brevets**

(11) Publication number : **0 245 119 B1**

## (12) EUROPEAN PATENT SPECIFICATION

(45) Date of publication of patent specification :
**25.03.92 Bulletin 92/13**

(51) Int. Cl.⁵ : **A61M 5/162**

(21) Application number : **87304138.8**

(22) Date of filing : **08.05.87**

(54) **Reverse taper spike connector.**

(30) Priority : **08.05.86 US 861022**

(43) Date of publication of application :
**11.11.87 Bulletin 87/46**

(45) Publication of the grant of the patent :
**25.03.92 Bulletin 92/13**

(84) Designated Contracting States :
**BE DE FR GB IT**

(56) References cited :
**EP-A- 0 093 837**
**EP-A- 0 157 224**
**FR-A- 2 097 975**

(73) Proprietor : **BAXTER INTERNATIONAL INC. (a
Delaware corporation)
One Baxter Parkway
Deerfield Illinois 60015 (US)**

(72) Inventor : **Bellotti, Marc
804 Furlong
Libertyville Illinois 60048 (US)**
Inventor : **Taylor, Larry C.
5421 Shore Drive
McHenry Illinois 60050 (US)**
Inventor : **Murphey, Randy K.
6628 125th Place
Kenosha Wisconsin 53142 (US)**

(74) Representative : **Bassett, Richard Simon et al
ERIC POTTER & CLARKSON St. Mary's Court
St. Mary's Gate
Nottingham NG1 1LE (GB)**

## Description

Field Of The Invention

The invention pertains to connectors usable to removably couple fluid flow conduits together. More particularly, the invention pertains to spike connectors and systems incorporating same for the purpose of providing relatively long term coupling between two different fluid flow conduits.

Background Of The Invention

Spike connectors are generally known. These connectors usually have a a grippable member to which is molded and outwardly extending, tapered spike. A fluid flow conduit extends through the grippable member and the spike. The spike is conventionally tapered such that it has a smaller diameter in a region located distally of the grippable member and a larger diameter in a region located proximally of the grippable member. The distal end of the spike is usually formed with a sharp, relatively pointed end. Such connectors may be readily inserted through pierceable membranes in containers filled with various types of medical fluids.

The containers are usually provided with a molded or extruded port in which the pierceable member is located. The taper of the spike connector provides for ready insertion into the port. Frictional forces are effective to keep the two connector members coupled together.

With certain procedures, particularly continuous ambulatory peritoneal dialysis (CAPD), it is not uncommon for a spike connector to be inserted into an access port of a dialysis solution container for 3 or 4 or more hours. Thus, there is a continuing need for a spike connector which is relatively highly resistant to separating from its mating connector. There is also a continuing need for a spike connector which is relatively easily insertable into the access port of a container.

Summary Of The Invention

FR-A-2097975 discloses a spike connector having a fluid flow path therethrough and comprising a grippable member and piercing means affixed to the member. The piercing means comprises an elongate body formed with a cylindrical portion having a hollow, piercing tip. A stop face is provided to limit penetration of the piercing means into a port, when the piercing means is inserted in the port to pierce a membrane in the port.

The pre-characterising part of Claim 1 is based on this disclosure and the invention is characterised in that a spike connector having a fluid flow path therethrough and comprising a grippable member, piercing means affixed to the member and comprising an elongate body formed with a cylindrical portion having a piercing tip, and a stop face whereby the piercing tip can be inserted in a tubular port to pierce a membrane therein with the stop face limiting penetration of the piercing means into the port, characterised in that the piercing means includes an elongate reverse tapered portion between the cylindrical portion and the stop face, reducing in diameter towards said stop face, whereby a peripheral wall of the port may be deformed, on penetration of the spike connector, to grip the reverse tapered portion and provide retaining forces to resist separation of the connector from the port.

An annular surface can be provided between a portion of the reversed tapered region and the cylindrical portion.

A second generally cylindrical region can be positioned between the grippable member and the reverse tapered region. The second generally cylindrical region may have a diameter greater than the diameter of the reverse tapered region.

The reverse tapered angle is preferably within a range of one-half to one degree when used with flexible plastic containers. A diameter of the reversed tapered region can be in a range of .200 to .220 inches (5.0 to 5.6 mm).

A connector system may include a spike connector as defined above and a connecting port having a hollow tubular body portion defining an elongated internal region closed by a pierceable membrane, the internal region including an entry part having an internal peripheral wall which is outwardly tapered, the piercing means of the spike connector being slidably engageable in the entry part to pierce the membrane and deform the peripheral wall to grip the reversed tapered portion and provide retaining forces to resist separation of the spike connector from the port.

The conformity between the portion of the peripheral wall and the reverse tapered region provides static frictional forces that, upon attempted separation of the connector members, oppose such separation. Further, as the static forces are overcome, and the connectors tend to move apart, a deflection of the peripheral wall is required to move the reversed tapered portion of the second connector therethrough. This results in a high dynamic retaining force.

The system may be for the delivery or receipt of selected medical fluids to or from a catheter which is implanted in a patient. The system then includes a container which is suitable for storage or receipt of a selected fluid. The container in provided with the port.

Fluid in the container can be delivered to the patient's catheter or fluid can be delivered from catheter to the container.

Numerous other advantages and features of the present invention will become readily apparent from the following detailed description of the invention and the embodiments thereof, from the claims, and from the accompanying drawings in which the details of the invention are fully and completely disclosed as a part of this specification.

## Brief Description Of The Drawings

FIGURE 1 is an enlarged fragmentary planar view of a prior art spike connector;

FIGURE 2 is an enlarged side elevational view of a spike connector having a reverse taper in accordance with the present invention;

FIGURE 3 is an enlarged planar bottom plan view of the spike connector having a reverse taper in accordance with the present invention;

FIGURE 4 is an enlarged fragmentary view of a portion of a reverse tapered spike connector in accordance with the present invention;

FIGURE 5 is an enlarged view, in section, of a mating connector usable with the reverse tapered spike connector of the present invention;

FIGURE 6 is an enlarged view, partially in section, illustrating the relationship between a reverse tapered spike connector and its mating connector subsequent to the two members being engaged;

FIGURE 7 illustrates a plurality of graphs of insertion force and withdrawal force with respect to several different spike connectors; and

FIGURE 8 is a partially fragmentary, schematic, view of a fluid delivery system in accordance with the present invention.

## Detailed Description

While this invention is susceptible of embodiment in many different forms, there is shown in the drawing and will be described herein in detail specific embodiments thereof with the understanding that the present disclosure is to be considered as an exemplification of the principles of the invention and is not intended to limit the invention to the specific embodiment illustrated.

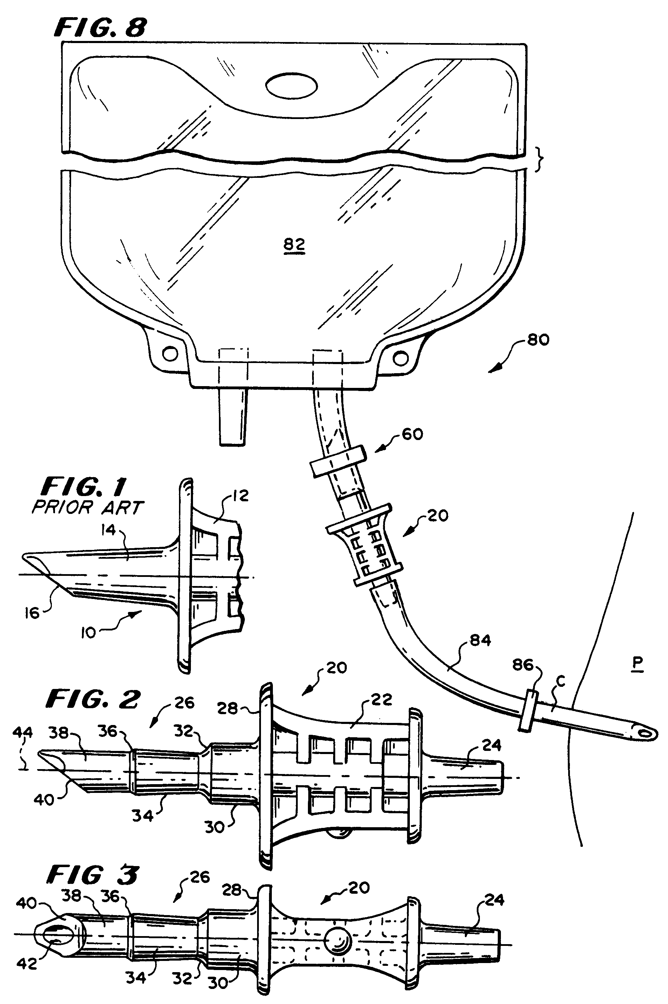

With respect to the figures, FIGURE 1 illustrates a prior art spike connector 10. The connector 10 includes a grippable member 12 to which is affixed a tapered piercing member 14. A fluid flow path extends through the member 14 and the member 12. The tapered piercing member 14 includes a pointed piercing end 16, whose purpose is to penetrate the sealing membrane of an accesss port of a container. The tapered piercing member 14 provides for ready insertion into an access port.

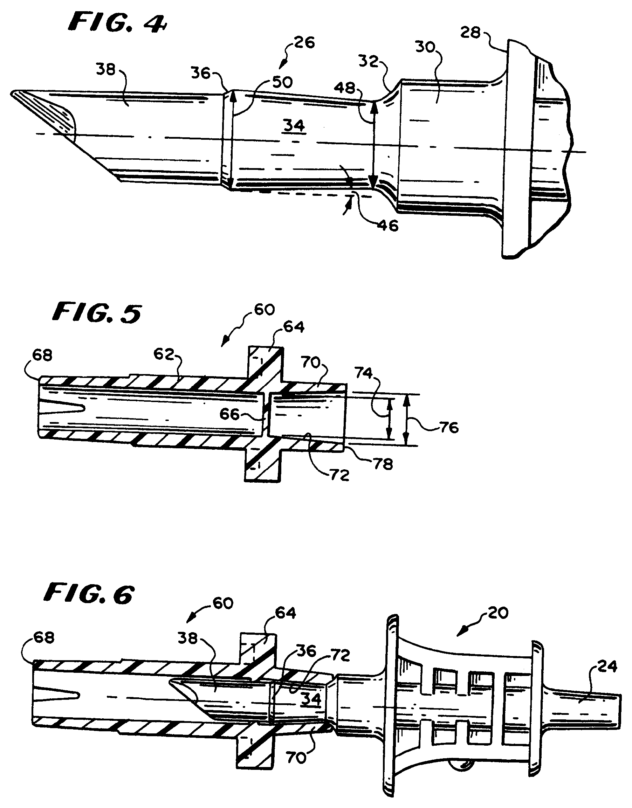

Figures 2 and 3 illustrate a spike connector 20 which has a reverse tapered region thereon. The connector 20 includes a grippable member 22, to which a fluid flow conduit may be affixed at a port 24. A multi-segment piercing member 26 is affixed to a surface 28 of the grippable member 22.

The piercing element 26 includes a cylindrical region 30 with a first end affixed to the surface 28. A second end 32 of the cylindrical region 30 is formed as an annular surface.

Extending from the annular surface 32 is a region 34 with a reverse taper formed thereon. For purposes of the present specification, the term "reverse taper" shall refer to and mean, as illustrated in Figures 2 and 3, a member having a region 34 with a diameter which is located proximally of the grippable member, which is less than a diameter which is located distally of the grippable member. The reverse taper region 34 terminates in a second annular surface 36.

Extending from the annular surface 36 is a generally cylindrical end 38 with a pointed piercing surface 40 thereon. A fluid flow path 42 is defined through the connector 20.

The members 30 and 34 of the piercing element 26 are symmetrical with respect to a center line 44 of the connector 20.

The grippable member 22 can assume a variety of shapes. It will be understood that the member 22 illustrated is exemplary only is not a limitation of the present invention.

The reverse taper spike connector 20 can be formed by injection molding of the element without the tapered region 34. The tapered region 34 can then be formed by machining the molded connector. The reverse taper spike connector 20 can be formed of an ABS-type medical grade plastic.

Figure 4 is an enlarged fragmentary view of the piercing member 26 illustrating the reverse tapered surface 34. The reverse tapered region 34 corresponds to an angle 46, which is in a range of one-half to one degree. Preferably, the angle 46 is on the order of .77 degree.

Figure 4 also illustrates that the reverse tapered member 34 includes a diameter 48 adjacent the annular surface 32, which is less than a diameter 50, which is adjacent the annular surface 36. The diameters 48 and 50 are preferably within a range of .200 to .220 inches (5.0 to 5.6 mm).

Figure 5 illustrates a mating port or connector 60, which is especially useful with the reverse tapered spike connector 20. The connector 60 is a molded connector with a generally cylindrical hollow body 62 with an integrally formed flange 64 thereon. The flange 64 provides rigidity and strength to the connector 62 in the region of an internal sealing membrane 66. In normal usage, the connector 60 would be affixed to a solution sealed container at an end 68. The membrane 66 would then provide a seal for the container.

The connector 60 also includes an access port at an end 70. The end 70 includes an internal peripheral wall 72, which is molded with an outward taper. For purposes of the present specification, the phraseology "outward taper" refers to the fact that the diameter 74 of the internal peripheral wall 72 adjacent the seal 66 is less than a diameter 76 of the internal peripheral wall adjacent an opening 78 of the end 70. The connector 60 is designed such that a reverse taper spike connector 20 can be inserted into the opening 78.

Figure 6 illustrates the reverse taper spike connector 20 inserted into the connector 60. As illustrated in Figure 6, the cylindrical region 38 of the spike connector 20 has pierced the membrane 66 providing access to the fluid in the associated container. Further, as can be seen from Figure 6, the internal peripheral wall 72 of the port region 70 conforms to and is in contact with, the reverse tapered region 34 of the connector 20. Further, the annular shoulder 36 is located adjacent an end of the flange 64.

As a result of the contact between the peripheral wall 72 and the reverse tapered region 34, when the spike connector 20 is to be withdrawn from the tubular connector 60, a substantial and high withdrawal force is present. This is because the annular surface 36 must now force apart the peripheral wall 72 during the removal process. This is a dynamic or drag frictional force which is present throughout the entire time the shoulder 36 is in contact with the peripheral wall 72.

It has been found that the minimum diameter 48 of the spike connector must exceed the minimum diameter 76 of the tubular connector 60 in order that the set or contact between the peripheral wall 72 and the reverse tapered region 34 will occur. The tubular connector 60 can be molded of an ABS medical grade plastic. Alternately, a polyvinyl chloride injection moldable material can also be used.

It has been noted that when the members 20 and 60 slidably engage one another, stresses are developed in the member 70 adjacent the flange 64. After the connector members have been engaged for a period of time, on the order of 3 to 4 hours, these stresses relax. As a result, the work necessary to separate the members 20 and 60 may be somewhat less after this period of time.

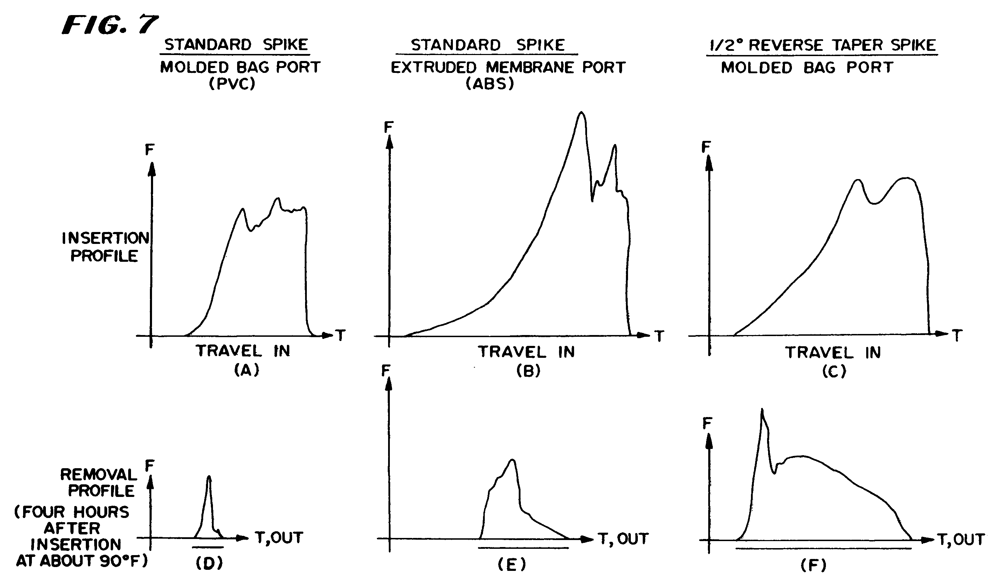

Figure 7 illustrates graphs of a variety of profiles of insertion and removal forces of spike connectors. Graph A illustrates the insertion force profile as a standard prior art spike, as in Figure 1, is inserted into a molded PVC-type port, such as the port 60. Figure B illustrates the force profile as a standard prior art spike, as in Figure 1, is inserted into an extruded port. Figure C illustrates the insertion force profile as a reverse tapered spike, as in Figures 2 and 3, is inserted into a molded connector, such as connector 60. As can be seen in Figure C, the insertion force is generally only slightly higher than in Figure A and generally slightly lower than in Figure B. The insertion force, therefore, is well within the acceptable range in the hands of a user.

Graph D of Figure 7 illustrates a force profile for the removal of a prior art tapered spike, as in Figure 1, from a molded connector, as the connector 60. Graph E illustrates the removal profile for a prior art spike, as in Figure 1, from an extruded port. As can be seen from Figure E, the removal forces in this instance are somewhat greater than in the case of Figure D. One of the reasons that the removal forces and the graph of Figure D are lower is that the taper of the prior art spike 10 closely matches the taper of the port 60, with respect to the internal peripheral surface 72.

Graph F illustrates the removal profile for a reverse tapered spike connector, such as the connector 20, from the molded port, such as the port 60. As can be seen from Figure F, there is a static force greater than those associated with Figures D and E which must be overcome before the connectors can start to move apart. Once the initial force is overcome, there is a continuing dynamic force due to the drag between the reverse tapered surface 34 and the interior peripheral surface 72, which has "set" against the reverse tapered surface. Thus, the reverse tapered spike connector 20, in connection with the connector member 60, provides for substantially greater retaining forces over a long period of time.

Figure 8 illustrates a fluid flow delivery system 80. The system 80 includes a container 82, a connector, such as the connector 60 affixed thereto, and a spike connector, such as the spike connector 20, engaged with the connector 60. The connector 20 is coupled by a fluid flow conduit 84 via a standard Luer-lock connector 86 to a catheter C which has previously been implanted in a patient P. For example and without limitation, the fluid flow system 80 could be usable in connection with continuous ambulatory peritoneal dialysis. In such an instance, dialysis fluid in the container 82 could be drained into the peritoneum of the patient P via the catheter C. The system 80 could then be worn by the patient for the dwell period, as has become conventional in connection with this modality. The high retaining forces between the spike connector 20 and the mating connector 60 are particularly desirable in this application, since the patient P might wear the system 80 for 3 or 4 more hours during the dwell period.

It will be understood by those skilled in the art that variations of the system 80 might make use of the reverse tapered spike connector 20. For example, depending on the material from which the mating connector, such as the connector 60, is formed, the taper angle 46 could be increased or decreased. Harder materials require less of a taper angle to achieve substantial retaining forces.

## Claims

1. A spike connector having a fluid flow path therethrough and comprising a grippable member (22), piercing means (26) affixed to the member and comprising an elongate body formed with a cylindrical portion (38) having a piercing tip (40), and a stop face (32) whereby the piercing tip can be inserted in a tubular port (60) to pierce a membrane (66) therein with the stop face limiting penetration of the piercing means into the port, characterised in that the piercing means (26) includes an elongate reverse tapered portion (34) between the cylindrical portion (38) and the stop face (32), reducing in diameter towards said stop face, whereby a peripheral wall (72) of the port may be deformed, on penetration of the spike connector, to grip the reverse tapered portion and provide retaining forces to resist separation of the connector from the port.

2. A spike connector as in Claim 1 including an annular surface (36) between the reverse tapered portion (34) and the cylindrical portion (38).

3. A spike connector as in Claim 1 or 2 including a second generally cylindrical region (30) positioned between said grippable member (22) and said reverse tapered portion (34).

4. A spike connector as in Claim 3 wherein said second cylindrical member 930) has a diameter greater than a diameter of said reverse tapered portion (34).

5. A spike connector as in Claim 4 wherein said second cylindrical member (30) has a diameter greater than any diameter of said reverse tapered portion (34).

6. A spike connector according to any preceding claim wherein said reverse tapered portion 934) corresponds to a taper angle in a range of one-half to one degree.

7. A spike connector as in Claim 6 wherein said taper angle substantially corresponds to .77 degree.

8. A spike connector as in any preceding claim wherein the diameter of said reverse tapered portion (34) is in a range of .200 to .220 inches (5.0 to 5.6 mm).

9. A spike connector according to any preceding claim in combination with and engaged with a tubular port (60).

10. A connector system comprising a spike connector according to any preceding claim and a connecting port (60) having a hollow tubular body portion (62) defining an elongated internal region closed by a pierceable membrane (66), said internal region including an entry part (70) having an internal peripheral wall (72) which is outwardly tapered, the piercing means (26) being slidably engageable in said entry part to pierce the membrane and deform the peripheral wall (72) to grip the reverse tapered portion (34) and provide retaining forces to resist separation of the spike connector from the connector port (60).

11. A connector system according to Claim 10, for delivery or receipt of selected medical fluids to or from a catheter implanted in a patient, comprising a container (82) suitable for storage or receipt of a selected fluid, wherein the connecting port (60) defines a pierceable access port to the container and the spike connector is coupled to a catheter (C).

12. A method of providing forces to retain the connector (20) and port (60) of any one of the preceding claims in a coupled relationship, comprising:

slidably inserting the reverse tapered portion (34), at least in part, into a region partly bounded by the interior peripheral wall (72) of the tubular port (60);

deflecting the interior peripheral wall (72) so that it contacts the reverse tapered portion (34) and assumes a corresponding taper; and

generating static and dynamic frictional forces opposing separation of the connector and port in response to the contact between the reverse tapered portion and the interior peripheral wall.


**Patentansprüche**

1. Spike-Verbindungsstück, durch das ein Fluidströmungsweg verläuft und das aufweist: ein greifbares Element (22), eine Durchstecheinrichtung (26), die an dem Element befestigt ist und einen langen Körper aufweist, an dem ein zylindrischer Abschnitt (38) mit einer Durchstechspitze (40) ausgebildet ist, und eine Anschlagfläche (32), wodurch die Durchstechspitze in eine rohrförmige Öffnung (60) einführbar ist, um eine darin angeordnete Membran (66) zu durchstechen, wobei die Anschlagfläche das Eindringen der Durchstecheinrichtung in die Öffnung begrenzt, dadurch gekennzeichnet, daß die Durchstecheinrichtung (26) zwischen dem zylindrischen Abschnitt (38) und der Anschlagfläche (32) einen langen umgekehrt konischen beschnitt (34) aufweist, dessen Durchmesser zur Anschlagfläche hin abnimmt, wodurch eine Umfangswand (72) der Öffnung bei Eindringen des Spike-Verbindungsstücks so verformbar ist, daß sie den umgekehrt konischen Abschnitt umgreift und Haltekräfte erzeugt, die einem Lösen des Verbindungsstücks von der Öffnung Widerstand entgegensetzen.

2. Spike-Verbindungsstück nach Anspruch 1, das zwischen dem umgekehrt konischen beschnitt (34) und dem zylindrischen Abschnitt (38) eine ringförmige Fläche (36) aufweist.

3. Spike-Verbindungsstück nach Anspruch 1 oder 2, das einen zweiten allgemein zylindrischen Bereich (30) aufweist, der zwischen dem greifbaren Element (22) und dem umgekehrt konischen Abschnitt (34) positioniert ist.

4. Spike-Verbindungsstück nach Anspruch 3, wobei der Durchmesser des zweiten zylindrischen Elements (30) größer als ein Durchmesser des umgekehrt konischen Abschnitts (34) ist.

5. Spike-Verbindungsstück nach Anspruch 4, wobei der Durchmesser des zweiten zylindrischen Elements (30) größer als jeder Durchmesser des umgekehrt konischen Abschnitts (34) ist.

6. Spike-Verbindungsstück nach einem der vorhergehenden Ansprüche, wobei der umgekehrt konische beschnitt (34) einem Konuswinkel in einem Bereich von 0,5-1° entspricht.

7. Spike-Verbindungsstück nach Anspruch 6, wobei der Konuswinkel im wesentlichen 0,77° entspricht.

8. Spike-Verbindungsstück nach einem der vorhergehenden Ansprüche, wobei der Durchmesser des umgekehrt konischen beschnitts (34) in einem Bereich von 5,0-5,6 mm (0,200-0,220 ") liegt.

9. Spike-Verbindungsstück nach einem der vorhergehenden Ansprüche in Kombination und in Verbindung mit einer rohrförmigen Öffnung (60).

10. Verbindungssystem, das ein Spike-Verbindungsstück nach einem der vorhergehenden Ansprüche und eine Verbindungsöffnung (60) mit einem hohlen rohrförmigen Körperteil (62) aufweist, der einen von einer durchstechbaren Membran (66) geschlossenen langen Innenbereich definiert, der einen Eintrittsteil (70) mit einer inneren Umfangswand (72) aufweist, die nach außen konisch verläuft, wobei die Durchstecheinrichtung (26) in den Eintrittsteil so einschiebbar ist, daß sie die Membran durchsticht und die Umfangswand (72) so verformt, daß diese den umgekehrt konischen beschnitt (34) umgreift und Haltekräfte erzeugt, die einem Lösen des Spike-Verbindungsstücks von der Verbindungsöffnung (60) Widerstand entgegensetzt.

11. Verbindungssystem nach Anspruch 10 zur Abgabe oder Aufnahme von ausgewählten medizinischen Fluiden zu oder von einem einem Patienten implantierten Katheter, wobei das Verbindungssystem einen zur Lagerung oder Aufnahme eines ausgewählten Fluids geeigneten Behälter (82) aufweist, wobei die Verbindungsöffnung (60) eine durchstechbare Zugangsöffnung zu dem Behälter definiert und das Spike-Verbindungsstück mit einem Katheter (C) gekoppelt ist.

12. Verfahren zur Erzeugung von Kräften, die das Verbindungsstück (20) und die Öffnung (60) nach einem der vorhergehenden Ansprüche miteinander gekoppelt halten, wobei das Verfahren folgende Schritte aufweist:

Einschieben des umgekehrt konischen beschnitts (34) wenigstens teilweise in einen von der inneren Umfangswand (72) der rohrförmigen Öffnung (60) teilweise begrenzten Bereich;

Auslenken der inneren Umfangswand (72) derart, daß sie den umgekehrt konischen beschnitt (34) berührt und eine entsprechende Konizität annimmt; und

Erzeugen statischer und dynamischer Reibungskräfte, die aufgrund des Kontakts zwischen dem umgekehrt konischen Abschnitt und der inneren Umfangswand einem Lösen des Verbindungsstücks von der Öffnung Widerstand entgegensetzen.

**Revendications**

1. Connecteur à aiguille à passage intérieur d'écoulement de fluide, comprenant une partie de prise (22), des moyens de perçage (26) fixés à cette partie et comportant un corps allongé qui présente une région cylindrique (38) à pointe de perçage (40), et une face de butée (32) de sorte que la pointe de perçage peut être insérée dans une tubulure (60) pour percer une membrane (66) fermant la tubulure, la face de butée limitant la pénétration des moyens de perçage dans la tubulure, caractérisé en ce que les moyens de perçage (26) comprennent une région allongée de conicité inversée (34) entre la région cylindrique (38) et la face de butée (32), dont le diamètre diminue vers ladite face de butée, de manière telle qu'une paroi périphérique (72) de la tubulure peut être déformée, lors de la pénétration du connecteur à aiguille, pour serrer la région de conicité inversée et engendrer des forces de retenue s'opposant à la séparation du connecteur et de la tubulure.

2. Connecteur à aiguille suivant la revendication 1, comprenant une surface annulaire (36) entre la région de conicité inversée (34) et la région cylindrique (38).

3. Connecteur à aiguille suivant la revendication 1 ou 2, comprenant une deuxième région sensiblement cylindrique (30) située entre ladite partie de prise (22) et ladite région de conicité inversée (34).

4. Connecteur à aiguille suivant la revendication 3, dans lequel ladite deuxième région cylindrique (30) a un diamètre plus grand qu'un diamètre de ladite région de conicité inversée (34).

5. Connecteur à aiguille suivant la revendication 4, dans lequel ladite deuxième région cylindrique (30) a un diamètre plus grand qu'un diamètre quelconque de la dite région de conicité inversée (34).

6. Connecteur à aiguille suivant l'une quelconque des revendications précédentes, dans lequel ladite région de conicité inversée (34) correspond à un angle de conicité compris entre 0,5 et 1 degré.

7. Connecteur à aiguille suivant la revendication 6, dans lequel ledit angle de conicité correspond sensiblement à 0,77 degré.

8. Connecteur à aiguille suivant l'une quelconque des revendications précédentes, dans lequel le diamètre de ladite région de conicité inversée(34) est compris entre 5,0 et 5,6 mm.

9. Connecteur à aiguille suivant l'une quelconque des revendications précédentes, en combinaison et en prise avec une tubulure (60).

10. Système de connexion comprenant un connecteur à aiguille suivant une quelconque des revendications précédentes et une tubulure de connexion (60) comportant un corps tubulaire creux (62) qui définit une région intérieure allongée fermée par une membrane perçable (66), ladite région intérieure comprenant une partie d'entrée (70) dont la paroi périphérique intérieure (72) est évasée vers l'extérieur, les moyens de perçage (26) pouvant s'engager de façon coulissante dans ladite partie d'entrée pour percer la membrane et déformer la paroi périphérique (72) de manière à serrer la région de conicité inversée (34) et à engendrer des forces de retenue s'opposant à la séparation du connecteur à aiguille et de la tubulure de connexion (60).

11. Système de connexion suivant la revendication 10 pour la distribution ou la réception de fluides médicaux choisis à destination ou en provenance d'un cathéter implanté dans un patient, comprenant un récipient (82) approprié au stockage ou à la réception d'un fluide choisi, dans lequel la tubulure de connexion (60) définit une tubulure d'accès perçable au récipient, et le connecteur à aiguille est raccordé à un cathéter (C).

12. Méthode de création de forces de retenue du connecteur (20) et de la tubulure (60) suivant l'une quelconque des revendications précédentes, dans une relation accouplée, comprenant :

l'insertion, de façon coulissante, de la région de conicité inversée (34),au moins en partie, dans une région partiellement délimitée par la paroi périphérique intérieure (72) de la tubulure (60) ;

la déformation de la paroi périphérique intérieure (72) de sorte qu'elle vienne en contact avec la région de conicité inversée (34) et prenne une conicité correspondante ; et

la génération de forces de frottement statiques et dynamiques s'opposant à la séparation du connecteur et de la tubulure, en réponse au contact entre la région de conicité inversée et la paroi périphérique intérieure.

**FIG. 8**

82

80

60

20

84

86

C

P

**FIG. 1**
**PRIOR ART**

12

14

16

10

**FIG. 2**

20

26

28

22

44

38

36

32

24

40

34

30

**FIG 3**

26

38

36

28

20

40

24

42

34

32

30

## FIG. 4

## FIG. 5

## FIG. 6

*FIG. 7*

STANDARD SPIKE
MOLDED BAG PORT
(PVC)

STANDARD SPIKE
EXTRUDED MEMBRANE PORT
(ABS)

1/2° REVERSE TAPER SPIKE
MOLDED BAG PORT

INSERTION PROFILE

F

TRAVEL IN
(A)

T

F

TRAVEL IN
(B)

T

F

TRAVEL IN
(C)

T

REMOVAL PROFILE

(FOUR HOURS AFTER INSERTION AT ABOUT 90°F)

F

(D)

T, OUT

F

(E)

T, OUT

F

(F)

T, OUT

10